Europäisches Patentamt

European Patent Office

Office européen des brevets

(19)

(11) Publication number: **0 139 356**

**A1**

(12) **EUROPEAN PATENT APPLICATION**

(21) Application number: **84304987.5**

(22) Date of filing: **23.07.84**

(51) Int. Cl.⁴: **A 61 F 2/34**
**A 61 F 2/46**

(30) Priority: **28.07.83 US 518271**

(43) Date of publication of application:
**02.05.85 Bulletin 85/18**

(84) Designated Contracting States:
**AT CH DE FR GB IT LI SE**

(71) Applicant: **Howmedica Inc.**
**235 East 42nd Street**
**New York New York 10017(US)**

(72) Inventor: **D'Errico, Joseph**
**11 Cresthill Avenue**
**Clifton New Jersey(US)**

(72) Inventor: **Turner, Roderick Habink**
**250 Beacon Street**
**Boston Massachusetts(US)**

(72) Inventor: **Scheller, Arnold Douglas, Jr.**
**181 Essex Street**
**North Quincy Massachusetts(US)**

(72) Inventor: **Cymbaluk, William John, Jr.**
**512 W. Scott Avenue**
**Rahway New Jersey(US)**

(72) Inventor: **Tatar, Stephen Lawrence**
**45 Upper Mountain Avenue**
**Montville New Jersey(US)**

(72) Inventor: **Campeau, Donald**
**419 La Salle Avenue**
**Hasbrouck Heights New Jersey(US)**

(74) Representative: **Graham, Philip Colin Christison et al,**
**Pfizer Limited Ramsgate Road**
**Sandwich, Kent CT13 9NJ(GB)**

(54) **Acetabular cup prosthesis.**

(57) An acetabular cup (10) for insertion into an acetabulum (16) comprises an outer metallic shell (12) and an inner resilient plastic insert (14), the insert (14) having a spherical shaped inner surface (20) adapted to articulate with a femoral head (22) and an outer surface (26) comprising a truncated cone (28) terminating at the upper end thereof in a spherical crown (30), the shell (12) having an inner surface (32) substantially congruent with the insert outer surface (26) and an essentially hemispherical outer surface (34) terminating in an annular flange (36) adapted to be countersunk into the acetabulum (16), the shell outer surface (34) having an annular ridge (38) of uniform height spaced from and parallel to the flange (36), the flange (36) and the ridge (38) being adapted to facilitate centering the cup (10) in the acetabulum (16) with the shell outer surface (34) uniformly spaced from the wall (40) of the acetabulum (16). Novel acetabular reamers (58,72) for use in installing the acetabular cup (10) are also disclosed.

_Fig.5._

PC 6729

## ACETABULAR CUP PROSTHESIS

This invention concerns an acetabular cup for a total hip joint prosthesis and its insertion within an acetabulum to provide a uniform spacing between the cup and its supporting bone member.

Numerous types of prostheses are available for use in hip joint repair or replacement, these prostheses generally employing an acetabular cup with a concave spherical surface and a femoral stem with a spherical joint head which fits into the cup. Currently, a major problem with acetabular cups during reconstructive surgery is the asymmetric placement of the cup within the natural acetabulum. This asymmetric placement produces an unequal layer of bone cement surrounding the cup, resulting in localized pressure points around the cup and early loosening of the cup from the acetabulum during subsequent use.

It is therefore a primary objective of the present invention to provide an acetabular cup prosthesis of improved stability with instrumentation for its insertion which readily and consistently places the prosthesis in the center of the acetabulum with an equispacing of cement all around the prosthesis.

A method of controlling the thickness of cement applied between a prosthetic insert and a support member by use of a cement spacer is disclosed in U.S. Patent 4,285,071. Prior acetabular cups having peripheral flanges and concentric ribbing are disclosed for example, in U.S. Patents 3,698,017, 4,031,570 and 4,141,088. An acetabular cup comprising a cup member encased in a metallic outer shell member is disclosed in U.S. Patent 4,172,296.

The desired objective is accomplished with an acetabular cup which comprises an outer metallic shell and an inner resilient plastic insert, the insert having a spherical shaped inner surface adapted to articulate with a femoral head and an outer surface comprising a truncated cone terminating at the upper end thereof in a spherical crown, the shell having an inner surface substantially congruent with the insert outer surface and an essentially hemispherical outer surface terminating in an annular flange adapted to be countersunk into an acetabulum. The shell outer surface has an annular ridge of uniform height spaced from and parallel to the flange, the flange and the ridge being adapted to facilitate centering the cup in the acetabulum with the shell outer surface uniformly spaced from the wall of the acetabulum.

Preferably, the insert outer surface and the shell inner surface are provided with congruent mating detent means to secure the insert within the shell. This detent means may include at least one projection on the insert outer surface to engage congruent indent means in the shell inner surface, the projections comprising a plurality of equispaced substantially rectangular ridges parallel to and adjacent the base of the insert. The detent means may also include at least one projection on the shell inner surface to engage congruent indent means in the insert outer surface, the projections comprising at least one semicylindrical protrusion parallel to the axis of the truncated cone to prevent rotation of the insert within the shell. The cup may further include an ileopsoas cutout at the mouth of the cup to preclude abrasion of the ileopsoas tendon by the cup.

Also provided are acetabulum reamers for use in installing the present acetabular cup, including a conventional hemispherical reamer with cutting teeth to denude the acetabulum. A second reamer comprises a smooth hemisphere girdled at the base thereof by a cutting flange provided with teeth for countersinking the periphery of the acetabulum for receipt of the cup flange to a depth limited by contact of the apex of the hemisphere with the bottom of the acetabulum. A third reamer comprises a hemisphere girdled at the base thereof by a smooth flange, the hemisphere being provided with cutting teeth to ream the acetabulum concentric with the smooth flange.

The above and other objects, features and advantages of the present invention will be apparent from the following detailed description of a preferred embodiment thereof in conjunction with the accompanying drawing wherein like reference numerals indicate like structure throughout the several views.

Figure 1 is a front elevational view of an acetabular cup prosthesis, according to the present invention;

Figure 2 is a top plan view of the cup partially broken away to illustrate a detent means;

Figure 3 is a bottom plan view of the cup;

Figure 4 is a side elevational view of the cup;

Figure 5 is a cross-sectional view taken along the line 5-5 of Figure 2 showing the cup inserted in the acetabulum;

Figure 6 is a front elevational view of the liner of the acetabular cup of Figure 1;

Figure 7 is a bottom plan view of the liner shown in Figure 6;

Figure 8 is a cross-sectional view thru the shell of the acetabular cup of Figure 1;

Figure 9 is an enlarged cross-sectional view showing a detent means during placement of the insert into the shell;

Figure 10 is a schematic diagram of a conventional hemispherical reamer within an acetabulum;

Figure 11 is a schematic diagram of a second reamer forming a countersunk area within the acetabulum; and

Figure 12 is a schematic diagram of a third reamer entering the countersunk acetabulum during its final stage of preparation.

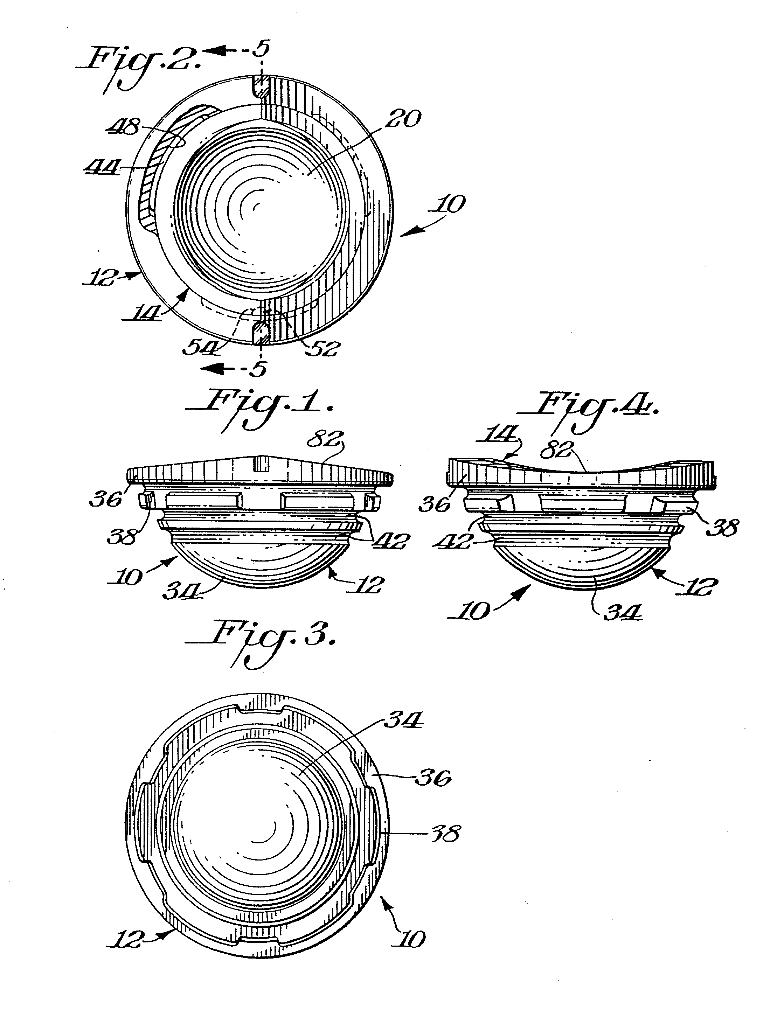

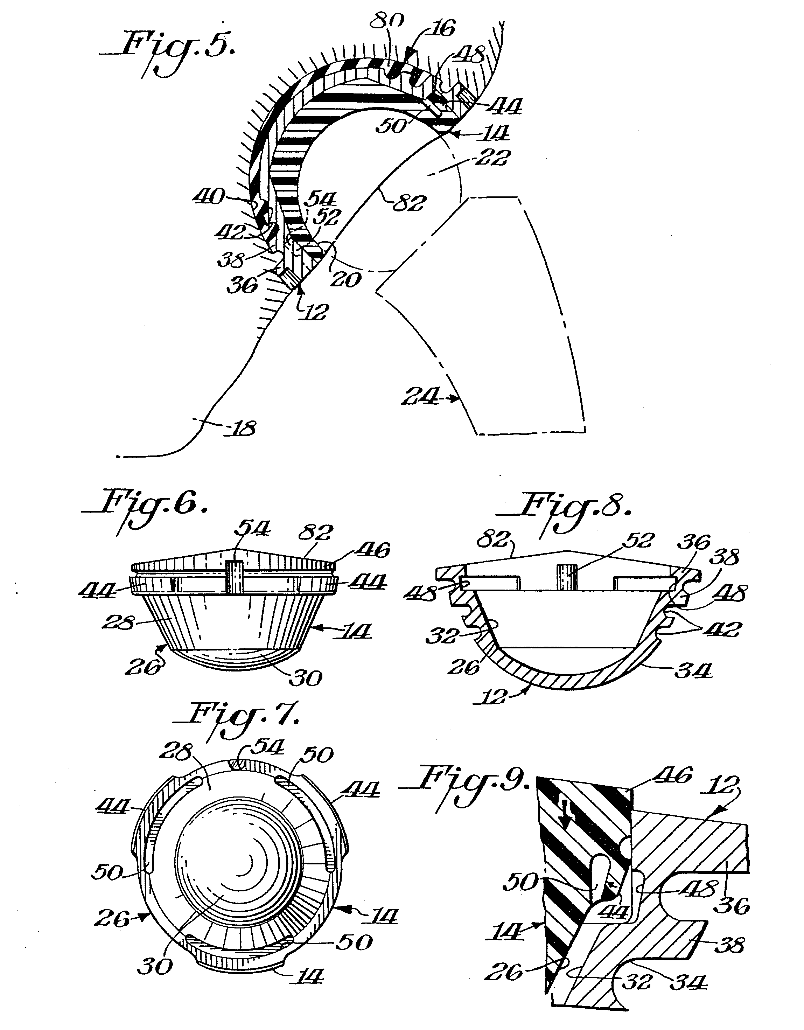

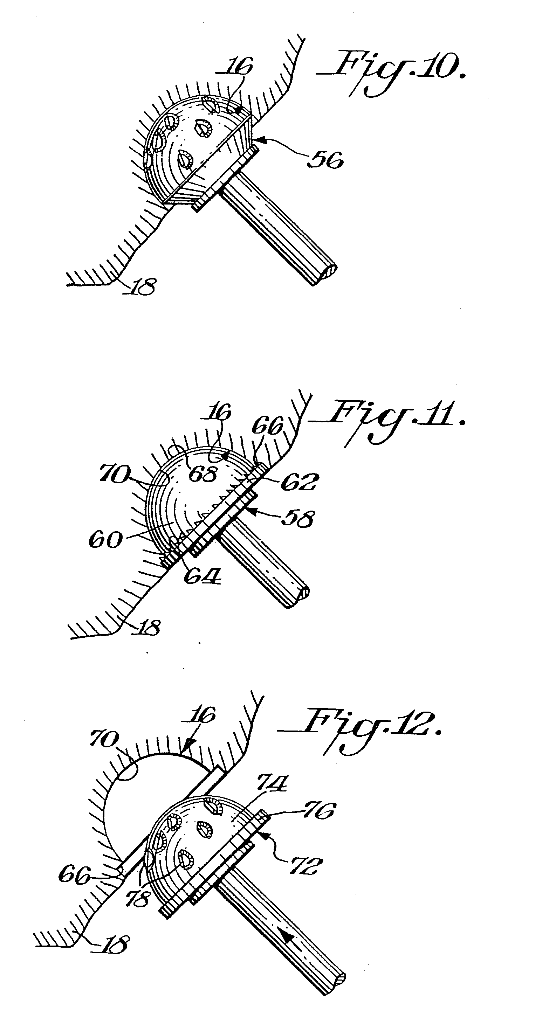

Referring to the drawing, Figures 1 thru 9 illustrate an acetabular cup 10 of a total hip replacement comprising an outer metallic shell 12 and an inner resilient plastic insert 14. The cup 10 serves as a replacement for the natural acetabulum cup and is constructed for introduction into an acetabulum 16 in the pelvis 18. The cup 10 may be used on either the right or the left of the pelvis 18, being introduced into the acetabulum 16 after appropriate preparation thereof. Such preparation requires reaming of the acetabulum 16 which, in the case of the present invention, includes a countersinking step as explained more fully below.

Insert 14, which may be of such as high density polyethylene, has a spherical shaped inner surface 20 which articulates with the spherical head 22 of a femoral prosthesis 24. The outer surface 26 of insert 14 comprises a truncated cone 28 terminating at the upper end in a spherical crown 30. Such shape for insert outer surface 26 provides improved distribution of compressive loading in the body and also simplifies the fabrication of the insert 14.

Shell 12, fabricated of such as cobalt/chromium/ molybdenum alloy, has an inner surface 32 substantially congruent with insert outer surface 26 and an essentially hemispherical outer surface 34 which terminates in an annular flange 36 adapted to be countersunk into acetabulum 16. Shell outer surface 34 also has an annular ridge 38 of uniform height spaced from and parallel to flange 36, the flange 36 and ridge 38 being adapted as explained below to facilitate the centering of cup 10 within the acetabulum 16 such that shell outer surface 34 is uniformly spaced from the wall 40 of acetabulum 16. Ridge 38 may be contiguous, but is preferably segmented to aid in the uniform flow of bone cement during placement of cup 10 in acetabulum 16. Shell outside surface 34 also includes two concentric, spaced undercuts 42 to help retain cup 10 within acetabulum 16.

Insert outer surface 26 and shell inner surface 32 are provided with congruent mating detent means to secure the insert 14 within shell 12. Such means include three equally spaced projections in the form of substantially rectangular ridges 44, parallel to and adjacent base 46 of insert 14, which engage congruent indents 48 in shell inner surface 32. Insert 14 is further provided with an undercut 50 behind each ridge 44, the function of undercut 50 being shown in Figure 9. As insert 14 is placed inside shell 12, undercut 50 allows ridge 44 to deform inward toward the center of insert 14 and thereby slip by shell 12. When insert 14 is fully within shell 12, ridge 44 snaps into indent 48 and thereby locks insert 14 securely within shell 12.

Shell 12 is also provided with a semicylindrical protrusion 52 parallel to the axis of truncated cone 28 which engages a congruent indent 54 on insert outer surface 26. This indent means combination of protrusion 52 and insert indent 54 not only provides an alignment guide upon the insertion of insert 14 into shell 12, but also prevents rotation of the insert 14 within shell 12 after the insertion.

The proper placement of cup 10 within acetabulum 16 requires a careful preparation of acetabulum 16. Such preparation will be described with the aid of Figures 10 thru 12.

Initially, as shown in Figure 10, acetabulum 16 is denuded with a conventional hemispherical reamer 56. Then, as shown in Figure 11, a second reamer 58, which comprises a smooth hemisphere 60 girdled at its base by a cutting flange 62 provided with teeth 64, planes the bone of pelvis 18 surrounding acetabulum 16 and thereby creates a countersunk area 66 to seat flange 36 of cup 10. The depth of such countersinking is limited by contact of the apex 68 of hemisphere 60 with the bottom 70 of acetabulum 16. A third reamer 72, shown in Figure 12, comprising a hemisphere 74 which is girdled at its base by a smooth flange 76 and is provided with teeth 78, finally reams acetabulum 16 concentric with flange 76.

This reaming preparation takes advantage of the configuration of shell 12 and provides a simple method of assuring symmetrical insertion of cup 10 within the acetabulum, the depth of insertion being limited by flange 36 abutting countersunk area 66 while sidewise movement is prevented by contact of ridge 38 with wall 40. The resulting uniform spacing 80 between cup 10 and wall 40 of typically about 2 millimeters insures uniform application of bone cement around cup 10, thereby minimizing localized stress.

Acetabulum cup 10 is of a low profile design to minimize removal of bone from acetabulum 16. The cup additionally includes an ileopsoas cutout 82 to prevent abrasion of the ileopsoas tendon (not shown) against cup 10.

While the invention has been described in connection with a preferred embodiment, it also includes alternatives, modifications and equivalents within the spirit and the scope of the appended claims.

## CLAIMS

1. An acetabular cup (10) comprising an outer metallic shell (12) and an inner resilient plastic insert (14) for insertion into an acetabulum (16), characterized in that

said insert (14) has a spherical shaped inner surface (20) adapted to articulate with a femoral head (22) and an outer surface (26) comprising a truncated cone (28) terminating at the upper end thereof in a spherical crown (30) and

said shell (12) has an inner surface (32) substantially congruent with said insert outer surface (26) and an essentially hemispherical outer surface (34) terminating in an annular flange (36) adapted to be countersunk into said acetabulum (16), with

said shell outer surface (34) having an annular ridge (38) of uniform height spaced from and parallel to said flange (36) and

said flange (36) and said ridge (38) being adapted to facilitate centering said cup (10) in said acetabulum (16) with said shell outer surface (34) uniformly spaced from the wall (40) of said acetabulum (16).

2. The cup (10) of claim 1 wherein said insert outer surface (26) and said shell inner surface (32) are provided with congruent mating detent means (44,48; 52,54) to secure said insert (14) within said shell (12).

3. The cup (10) of claim 2 wherein said detent means (44,48) include at least one projection (44) on said insert outer surface (26) to engage congruent indent means (48) in said shell inner surface (32).

4.  The cup (10) of claim 3 wherein said projections (44) comprise a plurality of equispaced substantially rectangular ridges parallel to and adjacent the base (46) of said insert (14).

5.  The cup (10) of claim 2 wherein said detent means (52,54) include at least one projection (52) on said shell inner surface (32) to engage congruent indent means (54) in said insert outer surface (26).

6.  The cup of claim 5 wherein said projections (52) comprise at least one semicylindrical protrusion parallel to the axis of said truncated cone (28) to prevent rotation of said insert (14) within said shell (12).

7.  The cup (10) of claim 1 including an ileopsoas cutout (82) at the mouth of said cup (10) to preclude abrasion of the ileopsoas tendon by said cup (10).

8.  An acetabulum reamer (58) for use in installing the cup (10) of claim 1, characterized in comprising a smooth hemisphere (60) girdled at the base thereof by a cutting flange (62) provided with teeth (64) for countersinking the periphery of said acetabulum (16) for receipt of said cup flange (36) to a depth limited by contact of the apex (68) of said hemisphere (60) with the bottom (70) of said acetabulum (16).

9.  An acetabulum reamer (72) for use in installing the cup (10) of claim 1, characterized in comprising a hemisphere (74) girdled at the base thereof by a smooth flange (76), with said hemisphere (74) being provided with cutting teeth (78) to ream said acetabulum (16) concentric with said smooth flange (76).

0139356

Fig.2.  ← 5

48

44

48

20

10

12

14

54    52
← 5

Fig.1.

82

36
38
10    34    42    12

Fig.4.

14    82

36
42
10    34    38    12

Fig.3.

34

36

38

12    10

*Fig.5.*

*Fig.6.*

*Fig.7.*

*Fig.8.*

*Fig.9.*

0139356

Fig.10.

16
56
18

Fig.11.

16    66
70    68    62
           58
60
64
18

Fig.12.

70    16
           74
           76
66    72
18
18

| | DOCUMENTS CONSIDERED TO BE RELEVANT | | EP 84304987.5 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | DE - A1 - 2 911 754 (H. REIMER) <br> * Fig. 1; claims 1,9; page 8, last paragraph * | 1 | A 61 F 2/34 <br> A 61 F 2/46 |
| A | US - A - 3 818 512 (Y.I. SHERSHER) <br> * Totality * | 1 | |
| A | DE - A1 - 2 950 536 (HOWMEDICA et al.) <br> * Fig. 2,4; pages 11-15 * | 1-3 | |
| A | US - A - 3 903 549 (W.M. DEYERLE) <br> * Totality * | 1,2,5 | |
| A | FR - A1 - 2 519 545 (J. CUILLERON) <br> * Totality * | 1-3 | TECHNICAL FIELDS SEARCHED (Int. Cl.4) |
| A | DE - B - 1 806 323 (W. LINK) <br> * Fig. 1; claim 1 * | 1,7 | A 61 F 1/00 <br> A 61 B 17/00 |
| A | DE - A1 - 2 830 566 (G. NEUHÄUSER PR.) <br> * Fig. 6; page 15, last paragraph - page 16, first paragraph * | 1,8 | |
| A | DE - B2 - 2 500 958 (H. WEIGAND et al.) <br> * Fig. 1; column 3, lines 45-54 * | 1,9 | |

The present search report has been drawn up for all claims

| Place of search | Date of completion of the search | Examiner |
|---|---|---|
| VIENNA | 12-12-1984 | LUDWIG |